# NEUE EUROPÄISCHE PATENTSCHRIFT

(11) **EP 2 674 479 B2**
(45) Veröffentlichungstag und Bekanntmachung des Hinweises auf die Entscheidung über den Einspruch: **12.03.2025**
(45) Hinweis auf die Patenterteilung: 18.03.2015
(21) Anmeldenummer: 12172304.3
(22) Anmeldetag: 15.06.2012
(51) Int. Cl.: C12M 1/02, B01L 1/04, B01F 7/16, B01F 13/08, B01F 15/06

(54) **Einwegbioreaktor und Kopfplatte sowie Herstellungsverfahren**
Disposable bioreactor and head plate and production method
Bioréacteur jetable et plaque frontale, ainsi que procédés de fabrication

(43) Veröffentlichungstag der Anmeldung: 18.12.2013
(73) Patentinhaber: Eppendorf SE, 22339 Hamburg (DE)
(72) Erfinder: Arnold, Matthias Dr.-Ing.,, 52074 Aachen (DE); Köhn, Heinz-Gerhard Dr. rer. nat.,, 22339 Hamburg (DE); Gülzow, Nico Dipl.-Ing.,, 22337 Hamburg (DE); Eikelmann, Sven Dipl.-Ing.,, 22299 Hamburg (DE); Selzer, Sebastian Dipl.-Ing., 40221 Düsseldorf (DE); Beese, Jochen, 22848 Norderstedt (DE); Günther, Christopher Dipl.-Ing.,, 22085 Hamburg (DE); Kopowski, Eckhart Dr.-Ing.,, 38102 Braunschweig (DE)
(74) Vertreter: Eisenführ Speiser

(56) Entgegenhaltungen:
- EP-A1- 2 065 085
- EP-A1- 2 065 085
- EP-A1- 2 251 407
- WO-A1-03/006633
- WO-A1-2009/009771
- WO-A2-2007/134267
- WO-A2-2008/088379
- WO-A2-2010/108091
- DE-A1- 102008 027 638
- DE-A1- 102009 056 468
- DE-U1- 202007 005 868
- DE-U1- 202009 015 434
- US-A1- 2007 253 288
- US-A1- 2008 131 957
- US-A1- 2009 152 744
- US-A1- 2011 058 447
- US-A1- 2011 058 448
- US-A1- 2011 207 170
- US-A1- 2012 003 733

## Beschreibung

Die Erfindung betrifft eine Kopfplatte für einen Einwegbioreaktor, insbesondere zur Verwendung in einem, vorzugsweise parallelen, Bioreaktorsystem, für die Anwendung in der Zellkultur und/oder der Mikrobiologie.

Die Erfindung betrifft ferner ein Verfahren zum Herstellen einer Kopfplatte für einen Einwegbioreaktor, insbesondere zur Verwendung in einem, vorzugsweise parallelen, Bioreaktorsystem, für die Anwendung in der Zellkultur und/oder der Mikrobiologie.

### Hintergrund der Erfindung

Bioreaktoren, die häufig auch als Fermenter bezeichnet werden, schließen einen Reaktionsraum ein, in dem biologische bzw. biotechnologische Prozesse im Labormaßstab durchgeführt werden können. Zu solchen Prozessen zählt beispielsweise die Kultivierung von Zellen, Mikroorganismen oder kleinen Pflanzen unter definierten, vorzugsweise optimierten, kontrollierten und reproduzierbaren Bedingungen. Bioreaktoren verfügen dazu meist über mehrere Anschlüsse, über die Primär- und Sekundärstoffe sowie verschiedene Instrumente, wie beispielsweise Sensoren, in den Reaktionsraum eingebracht werden können oder über die beispielsweise Fluidleitungen, insbesondere Gasleitungen, wie Begasungs- oder Abgasleitungen, angeschlossen werden können. Bioreaktoren weisen ferner in der Regel ein Rührwerk auf, dessen Rührwelle von einem Antrieb in Rotation versetzt werden kann, wodurch ein drehsteif mit der Rührwelle verbundenes Rührelement ebenfalls in Rotation versetzt wird und so eine Durchmischung der im Reaktionsraum vorhandenen Stoffe bewirkt. Es können auch zwei oder mehr Rührelemente, meist axial beabstandet, auf der Rührwelle angeordnet und mit dieser verbunden sein. Das Rührelement bzw. die Rührelemente kann bzw. können auch einstückig mit der Rührwelle ausgebildet sein.

Sowohl für den Anwendungsbereich der Zellkultivierung als auch für den mikrobiologischen Anwendungsbereich ist die Verwendung von Bioreaktoren in, vorzugsweise parallelen, Bioreaktorsystemen bevorzugt. Parallele Bioreaktorsysteme sind beispielsweise in der DE 10 2011 054 363.5 oder der DE 10 2011 054 365.1 beschrieben. In einem solchen Bioreaktor-System können mehrere Bioreaktoren parallel betrieben und mit hoher Genauigkeit kontrolliert werden. Dabei können auch bei kleinen Arbeitsvolumina in den einzelnen Bioreaktoren Experimente mit hohem Durchsatz durchgeführt werden, die gut reproduzierbar und skalierbar sind. Der Labormaßstab von Bioreaktoren, auf die sich die Erfindung bezieht, liegt etwa bei einer Größe von bis zu 2000 ml, beispielsweise bei einem Gesamtvolumen des Reaktionsraums von etwa 350 ml bei einem Arbeitsvolumen von etwa 60 bis etwa 250 ml.

Im Anwendungsbereich der Zellkultur werden solche parallelen Bioreaktorsysteme beispielsweise für auf statistischen Planungsmethoden (Design of Experiments DoE) basierenden Versuchsreihen zur Prozessoptimierung, die Prozessentwicklung sowie die Forschung und Entwicklung eingesetzt, beispielsweise um verschiedene Zelllinien, wie Chinese Hamster Ovary (CHO)-, Hybridoma- oder NSO-Zelllinien, zu kultivieren. Unter dem Begriff "Zellkultur" wird im Rahmen des vorliegenden Textes insbesondere die Kultivierung tierischer oder pflanzlicher Zellen in einem Nährmedium außerhalb des Organismus verstanden.

Im Anwendungsbereich der Mikrobiologie werden parallele Bioreaktorsysteme ebenfalls für auf statistischen Planungsmethoden (Design of Experiments DoE) basierenden Versuchsreihen zur Prozessoptimierung, die Prozessentwicklung sowie die Forschung und Entwicklung eingesetzt, beispielsweise um verschiedene Mikroorganismen, insbesondere Bakterien oder Pilze, z.B. Hefen, zu kultivieren.

Aufgrund von meist begrenztem Platz im Labor werden dabei geringe Platzanforderungen, insbesondere geringe Stellplatzanforderungen sowohl für Bioreaktorsysteme als auch für Bioreaktoren selbst angestrebt.

Bioreaktoren im Laboreinsatz sind oft aus Glas und/oder Metall, insbesondere rostfreiem Stahl, ausgebildet, da die Bioreaktoren zwischen verschiedenen Anwendungen sterilisiert werden müssen, was vorzugsweise durch eine Heißdampfsterilisation in einem Autoklaven erfolgt. Die Sterilisierung und Reinigung wieder verwendbarer Bioreaktoren ist aufwändig: Der Sterilisations- und Reinigungsprozess kann einer Validierung unterliegen und seine Durchführung ist für jeden einzelnen Bioreaktor genau zu dokumentieren. Rückstände in einem nicht vollständig sterilisierten Bioreaktor können die Ergebnisse eines nachfolgenden Prozesses verfälschen oder unbrauchbar machen und einen nachfolgenden Prozessablauf stören. Des Weiteren können einzelne Bestandteile bzw. Materialien der Bioreaktoren durch den Sterilisationsprozess beansprucht und z.T. beschädigt werden.

Eine Alternative zu wieder verwendbaren Bioreaktoren stellen Einwegbioreaktoren dar, die für die Durchführung lediglich eines biologischen bzw. biotechnologischen Prozesses verwendet und anschließend entsorgt werden. Durch die Bereitstellung eines neuen, vorzugsweise im Herstellungsprozess sterilisierten, Einwegbioreaktors für jeden Prozess kann die Gefahr einer (Kreuz-)Kontamination reduziert werden und gleichzeitig entfällt der Aufwand der Durchführung und Dokumentation einer einwandfreien Reinigung und Sterilisierung eines zuvor genutzten Bioreaktors. Einwegbioreaktoren sind oft als flexible Behälter ausgebildet, beispielsweise als Beutel oder als Behälter mit zumindest abschnittsweise flexiblen Wänden. Beispiele für solche Bioreaktoren sind in US 2011/0003374 A1, US2011/0058447A1, DE 20 2007 005 868U1, US 2011/0058448A1, US2011/0207218A1, WO 2008/088379A2, US 2012/0003733 A1, WO2011/079180A1, US2007/0253288A1, US 2009/0275121A1 und US 2010/0028990A1 beschrieben. In der DE 20 2007 005 868 U1 z.B. ist ein Bioreaktor beschrieben mit flexiblen Wänden und mit einem von einer Seitenwandung, einem Bodenteil und einem Deckenteil umgrenzten Reaktorinnenraum, in dem mindestens ein Mischer angeordnet ist, der von einem außerhalb des Reaktorinnenraumes angeordneten Antrieb antreibbar ist. Die WO 2008/088379 A2 beschreibt Umgebungseinschließungssysteme, und in bestimmten Ausführungsformen Systeme und Verfahren, die Behältnisse oder andere Vorrichtungen umfassen, die umgebungseinschließend ausgerüstet sind, wobei die Behältnisse oder Vorrichtungen ausgebildet sein können, Fluide zu handhaben und/oder chemische, biochemische und/oder biologische Prozesse ausführen zu können. Diese Einwegreaktoren mit flexiblen Wänden haben jedoch unter anderem den Nachteil, dass sie in parallelen Bioreaktorsystemen, die für formstabile, wieder verwendbare Bioreaktoren ausgelegt sind, nicht verwendet werden können.

Ferner sind aus Gründen der Übertragbarkeit von Prozessen auf andere Maßstäbe unter anderem auch qualitativ vergleichbare und insbesondere definierte Strömungsverhältnisse (insbesondere bei der Durchmischung) im Kultivierungsraum wichtig. Diese Anforderung kann bei Bioreaktoren mit flexiblen Wänden nicht bzw. nur mit aufwändigen Zusatzmaßnahmen gewährleistet werden.

Formstabile Einwegreaktoren sind beispielsweise aus der EP 2 251 407 A1 und der US 2009/0311776 A1 bekannt. Die EP 2 251 407 A1 offenbart einen Einweg-Bioreaktor, der aus einem Behälter und einem Deckel besteht, wobei der Deckel mit dem Behälter verbindbar ist und nicht zerstörungsfrei entfernt werden kann. Am Markt erhältliche formstabile Einwegbioreaktoren sind beispielsweise Celligen Blu, Millipore Mobius und Sartorius UniVessel. Diese bekannten formstabilen Einwegbioreaktoren sind jedoch einerseits hochpreisig und andererseits von ihrer Konstruktion auf die pharmazeutische Prozessentwicklung und pharmazeutische Produktionsprozesse abgestimmt. Sie werden insbesondere für Zellkulturprozesse verwendet und sind demnach auch insbesondere auf solche Zellkulturprozesse konstruiert und abgestimmt. Für Anwendungen in der Mikrobiologie gelten jedoch andere Anforderungen, sowohl was die am Markt erzielbaren Preise als auch geeignete Konstruktion und einsetzbare Werkstoffe angeht, um den um Größenordnungen höheren Anforderungen an prozesstechnische Parameter, wie beispielsweise Mischzeit, Energieeintrag und Gasaustausch, gerecht zu werden. Die bekannten formstabilen Einwegbioreaktoren sind daher für eine Verwendung beispielsweise in der mikrobiologischen Forschung und Prozessentwicklung nicht geeignet.

Es ist daher eine Aufgabe der vorliegenden Erfindung, eine Kopfplatte bereitzustellen und Herstellungsverfahren anzugeben, die einen oder mehrere der genannten Nachteile verringern oder beseitigen. Eine weitere Aufgabe der Erfindung ist es, eine Kopfplatte bereitzustellen, die kostengünstig herzustellen sind sowie einfache und kostengünstige Herstellungsverfahren für eine Kopfplatte anzugeben.

### Zusammenfassung der Erfindung

Diese Aufgabe wird gelöst durch eine Kopfplatte nach Anspruch 1 und ein Verfahren zum Herstellen einer Kopfplatte nach Anspruch 11. Die Kopfplatte ist insbesondere geeignet für einen im folgenden beschriebenen Einwegbioreaktor, seine verschiedenen Aspekte und Fortbildungen.

Gemäß einem ersten Aspekt ist ein Einwegbioreaktor der eingangs genannten Art dadurch gekennzeichnet, dass das Rührwerk und das Lager vollständig in dem Reaktionsraum angeordnet sind und die Rührwelle einen magnetischen Abschnitt aufweist, der derart angeordnet und ausgebildet ist, dass er in einer axialen Richtung mit einem Rotationsantrieb magnetisch koppelbar ist. Da der Rotationsantrieb das Rührwerk mit einer Rührwelle und einem Rührelement antreibt, wird er auch als Rührantrieb bezeichnet.

Dieser Einwegbioreaktor zeichnet sich unter anderem dadurch aus, dass das Rührwerk durch einen Magnetantrieb in Rotation versetzt werden kann. Ein Vorteil dieser Ausbildung liegt darin, dass sowohl das Rührwerk als auch das Lager vollständig in dem Reaktionsraum angeordnet sind, d.h. dass keine Durchführung der Rührwelle durch die Kopfplatte erforderlich ist. Auf diese Weise ist auch keine Abdichtung einer solchen Durchführung der Rührwelle durch die Kopfplatte erforderlich. Dies hat den Vorteil, dass die Sterilität des Reaktionsraums nicht durch eine unzureichende Abdichtung einer Durchführung in der Kopfplatte beeinträchtigt werden kann. Ein weiterer Vorteil ist, dass die bei Konstruktionen mit abgedichteter Durchführung auftretenden Reibungswiderstände, die unter anderem zu einer Beschädigung von Septumkomponenten führen können, vollständig vermieden werden.

Ferner ist vorgesehen, dass eine Kopplung zwischen einem magnetischen Abschnitt der Rührwelle und einem außerhalb des Reaktionsraums an der Außenseite der Kopfplatte angeordneten Antrieb über eine Magnetkopplung in axialer Richtung, d.h. in Richtung der Rotationsachse oder parallel dazu, erfolgt. Zwischen einem magnetischen Abschnitt der Rührwelle und einem magnetischen Antriebsabschnitt des Rotationsantriebs ist vorzugsweise ein minimaler Luftspalt ausgebildet. Eine solche stirnseitige magnetische Kopplung zwischen Rührwelle und Rotationsantrieb hat gegenüber einer Kopplung in radialer Richtung, bei der ein magnetischer Abschnitt des Rotationsantriebs außenumfänglich um einen magnetischen Abschnitt an der Rührwelle angeordnet und beispielsweise in der US 2011/0058447 A1 beschrieben ist, den Vorteil, dass auf der Kopfplatte nur ein geringer Platz für den Rotationsantrieb erforderlich ist. Ein Rotationsantrieb mit einer stirnseitigen Magnetkopplung kann beispielsweise im Wesentlichen zylinderförmig aufgebaut sein, wobei die Querschnittsfläche des Zylinders im Wesentlichen der für die stirnseitige Magnetkopplung erforderlichen Fläche entsprechen kann. Auf diese Weise bleibt auf der Kopfplatte mehr Raum, um weitere Anschlüsse für Instrumente, Sensoren oder Funktionselemente anzuordnen.

Zur Aufnahme des magnetischen Abschnitts der Rührwelle kann die Kopfplatte beispielsweise eine Ausbuchtung aufweisen, die beispielsweise einen kreisförmigen Querschnitt haben kann und die beispielsweise ein außerhalb des Reaktionsraums angeordneter Rotationsantrieb mit einem Ring umgreifen kann, um den Rotationsantrieb konzentrisch über der Ausbuchtung und dem darin vorzugsweise ebenfalls konzentrisch angeordneten magnetischen Abschnitt der Rührwelle anzuordnen. Der magnetische Abschnitt der Rührwelle ist vorzugsweise an einem Ende der Rührwelle angeordnet ist und weist vorzugsweise einen größeren Durchmesser bzw. einen größeren Umfang auf als ein übriger Abschnitt der Rührwelle.

In einer bevorzugten Ausführungsform ist der magnetische Abschnitt einstückig mit der Rührwelle ausgebildet. Dies hat den Vorteil, dass die Anzahl an Teilen im Reaktionsraum reduziert wird und damit auch mögliche Spalte und Toträume zwischen verschiedenen Teilen reduziert werden.

Besonders bevorzugt ist es, dass der magnetische Abschnitt aus einem Kompositwerkstoff mit einer Kunststoffmatrix und einem magnetischen Material besteht oder einen solchen Kompositwerkstoff aufweist. Die hat den Vorteil, dass eine Materialkombination gewählt werden kann, die den Anforderungen der United States Pharmacopeia (USP) Klasse VI entspricht, beispielsweise durch Auswahl eines entsprechend klassifizierten Kunststoffes als Matrix.

Ferner ist bevorzugt, dass der magnetische Abschnitt durch Aufspritzen des Zweikomponentenwerkstoffs mit einer Magnetkomponente auf die Rührwelle hergestellt ist.

Durch Aufspritzen eines solchen Kunststoff-Magnet-Gemischs als Kompositwerkstoff, vorzugsweise im Spritzgießverfahren, auf ein Ende der Rührwelle kann eine besonders einfache und günstige Herstellung der Rührwelle mit stirnseitigem Magnetkopplungsabschnitt erzielt werden, die durch die einstückige Ausbildung gleichzeitig die Anzahl der Teile im Reaktionsraum reduziert.

In einer besonders bevorzugten Ausführungsform weist der magnetische Abschnitt einen Querschnitt in einer Ebene orthogonal zur Rührwelle auf, und weist über den Großteil dieses Querschnitts, vorzugsweise über den gesamten Querschnitt, eine magnetische Kraftwirkung für eine magnetische Kopplung in axialer Richtung mit einem Rotationsantrieb auf.

Eine solche Ausgestaltung eines vorzugsweise flächigen, zusammenhängenden Querschnitts des Bereichs mit magnetischer Kraftwirkung kann insbesondere durch Spritzgießen eines Kompositwerkstoffs mit einer Kunststoffmatrix und einem magnetischen Material in einer Spritzgießform erzielt werden, die ihrerseits einen Magneten aufweist, um das magnetischen Material des Kompositwerkstoffs beim Spritzgießen auszurichten. Der Querschnitt des magnetischen Abschnitts mit magnetischer Kraftwirkung ist vorzugsweise kreisförmig, ellipsenförmig oder rechteckig. Innerhalb dieses Querschnitts des magnetischen Abschnitts mit magnetischer Kraftwirkung sind vorzugsweise Segmente unterschiedlicher Polung ausgebildet. Beispielsweise können die Segmente ein sternförmiges Muster oder ein Muster mit tortenstückförmigen Segmenten bilden.

Eine solche Ausgestaltung hat mehrere Vorteile: Zum einen kann gegenüber einer Ausbildung mit mehreren, ringförmig angeordneten Stabmagneten ein höheres Drehmoment übertragen werden, was insbesondere erforderlich ist zur Erzielung hoher Drehzahlen von über 1500 rpm, insbesondere bis zu 2000 rpm, bis zu 3000 rpm oder darüber, die insbesondere für Anwendungen in der Mikrobiologie erforderlich sind. Dabei kann gleichzeitig die erforderliche Querschnittsfläche am Ende der Rührwelle und damit der erforderliche Bauraum auf der Kopfplatte gering gehalten werden. Ferner kann durch die Ausbildung von bestimmten Polsegmentmustern eine spezifische Passung von Bioreaktoren zu einem entsprechend ausgebildeten Rotationsantrieb erzielt und damit die Prozesssicherheit erhöht werden, da nur Bioreaktoren mit passender Polsegmentierung von einem Rotationsantrieb angetrieben werden können. Dies ist vorteilhaft, um den Antrieb mit dem gewünschten Drehmoment und der gewünschten Drehzahl sicherzustellen, da Drehmoment und insbesondere erzielte Drehzahl nur bei mechanisch an einen Rotationsantrieb gekoppelten Rührwerken überwacht werden, nicht hingegen bei magnetischen Kopplung von Rührwerk und Rotationsantrieb. Eine abweichende Drehzahl bei Verwendung beispielsweise eines Rotationsantriebs mit zu geringer Leistung kann jedoch den Kultivierungsprozess negativ beeinflussen.

Auch in dieser Ausgestaltung kann der magnetische Abschnitt einstückig mit, vorzugsweise einem Ende, der Rührwelle verbunden sein, beispielsweise durch Aufspritzen des Kompositwerkstoffs auf die Rührwelle. Alternativ kann der magnetische Abschnitt als separates Teil, vorzugsweise im Spritzgussverfahren, ausgebildet und an der Rührwelle angeordnet sein.

Gemäß einem zweiten Aspekt ist ein eingangs genannter Einwegbioreaktor oder ein Einwegbioreaktor gemäß dem ersten Aspekt dadurch gekennzeichnet, dass das Lager als Wälzlager ausgebildet ist.

Die in bekannten formstabilen Einwegbioreaktoren für die Anwendung in der Zellkultur eingesetzten Gleitlagerungen haben den Nachteil, dass die Drehzahlen des Rührwerks auf Bereiche von etwa 500 rpm beschränkt sind, da höhere Drehzahlen durch eine starke Reibung der Welle und des Lagers Abwärme erzeugen, die zu einem Schmelzen der Lagerung und damit zu einem Stillstand der Welle führen kann, was den Kultivierungsprozess beenden würde. Eine Gleitlagerung hat den weiteren Nachteil eines erhöhten Material-, insbesondere Kunststoffabriebs, der im Reaktionsraum einen unerwünschten Agglomerationskeim für Zellen darstellen würde und daher beispielsweise in einem Lagergehäuse aufgefangen werden muss.

Mit der Ausbildung der Lagerung der Rührwelle als Wälzlager können deutlich höhere Drehzahlen von über 1500 rpm, insbesondere bis zu 2000 rpm, bis zu 3000 rpm oder darüber, realisiert werden, die insbesondere für Anwendungen in der Mikrobiologie erforderlich sind.

Insbesondere ist bevorzugt, dass das Wälzlager als Polymerkugellager mit Glaskugeln ausgebildet ist, wobei das Polymerkugellager vorzugsweise einen Käfig aus einem thermoplastischen Kunststoff aufweist, beispielsweise aus Polyethylen, Polypropylen, Polyvinylidenfluorid, Polyetheretherketon oder Polytetrafluorethylen. Diese Materialpaarung für das Wälzlager hat einerseits den Vorteil, dass die Glaskugeln im Polymerkugellager ruhig und leise laufen und damit die Geräuschbelastung im Labor senken können. Ferner ist die Materialpaarung beständig gegenüber hochmolaren Laugen und Basen, die insbesondere bei mikrobiologischen Anwendungen zum Einsatz kommen und daher entsprechende Anforderungen an die Konstruktion und die eingesetzten Werkstoffe stellen.

Gemäß einem dritten Aspekt ist der eingangs genannte Einwegbioreaktor oder einer der zuvor beschriebenen Einwegbioreaktoren nach dem ersten oder zweiten Aspekt dadurch gekennzeichnet, dass die Kopfplatte und der Behälter unlösbar miteinander verbunden sind, die Kopfplatte aus einem ersten Material und der Behälter aus einem zweiten Material ausgebildet ist und die Rührwelle und/oder das Rührelement aus einem dritten Material ausgebildet ist bzw. sind, wobei das erste Material und das zweite Material eine höhere Temperaturbeständigkeit aufweisen als das dritte Material.

Bei diesem Einwegbioreaktor sind die Kopfplatte und der Behälter unlösbar miteinander verbunden, beispielsweise durch ein stoffschlüssiges Fügeverfahren wie Schweißen. Besonders bevorzugt ist eine Verbindung durch Ultraschallschweißen, da dieses Verfahren eine hohe Prozesssicherheit aufweist und zu einer sehr guten Abdichtung der Verbindung zwischen Kopfplatte und Reaktionsraum und damit zu einer guten Abdichtung des Reaktionsraums gegenüber der Umgebung führt. Ultraschallschweißen ist darüber hinaus ein sehr schnelles Verbindungsverfahren und reduziert den Fertigungsaufwand.

Eine unlösbare Verbindung von Kopfplatte und Behälter hat den Vorteil, dass nach einer Sterilisation im Herstellungsprozess der Einwegbioreaktor nicht mehr geöffnet werden kann und somit das Risiko einer Kontamination des Reaktionsraums vor der Nutzung des Einwegbioreaktors vermindert werden kann. Ferner hat diese unlösbare Verbindung den Vorteil, dass der Einwegbioreaktor auch nach der Nutzung nicht geöffnet werden kann und seine geschlossene, formstabile Ausbildung beibehält.

Insbesondere im Zusammenhang mit der Dekontaminierung von benutzten Einwegbioreaktoren werden auch die Vorteile der Materialpaarung deutlich: Einwegbioreaktoren aus Materialen mit einer geringeren Temperaturbeständigkeit haben den Nachteil, dass sie bei einer Dekontaminierung bzw. post-process Sterilisation beschädigt werden, z.B. teilweise schmelzen und daher in einer, vorzugsweise temperaturbeständigen, Umverpackung sterilisiert werden müssen. Die Ausbildung von Kopfplatte und Behälter aus Materialien mit einer Temperaturbeständigkeit, die eine Dekontaminierung erlauben, d.h. die bei einer Dekontaminierung nicht zerstört oder nennenswert angegriffen werden, hat den Vorteil, dass der Einwegbioreaktor beispielsweise mittels Dampfsterilisation dekontaminiert werden kann, ohne dass der Einwegbioreaktor dazu zusätzlich verpackt oder in einem weiteren Behältnis angeordnet werden müsste, da Kopfplatte und Behälter diesem Dekontaminierungsprozess standhalten.

Durch die Kombination mit einer Rührwelle und/oder einem Rührelement aus einem Material mit geringerer Temperaturbeständigkeit wird der Vorteil erreicht, dass beim Dekontaminationsprozess die Rührwelle und/oder das Rührelement zerstört oder zumindest funktionsuntüchtig gemacht werden. Das dritte Material weist dazu entsprechende Eigenschaften auf. Insbesondere ist eine Temperaturbeständigkeit des dritten Materials bevorzugt, die einem Dekontaminierungsprozess nicht standhalten.

Auf diese Weise kann zuverlässig eine gewollte oder versehentliche Wiederverwendung eines bereits einmal benutzten Einwegbioreaktors verhindert werden, da das Rührwerk nach der Dekontaminierung nicht mehr verwendet werden kann und gleichzeitig durch die unlösbare Verbindung von Kopfplatte und Behälter der Einwegbioreaktor auch nicht geöffnet werden kann, um das Rührwerk auszutauschen.

Ferner ist besonders bevorzugt, dass das erste Material der Kopfplatte und das zweite Material des Behälters gleich sind, was insbesondere von Vorteil ist, wenn als Fügeverfahren zum Verbinden von Kopfplatte und Behälter Ultraschallschweißen eingesetzt wird.

Die Temperaturbeständigkeit von Materialien wird hier vorzugsweise als deren Glasübergangstemperatur definiert.

Besonders bevorzugt ist, dass das erste Material und das zweite Material eine höhere Glasübergangstemperatur aufweisen als das dritte Material. Die Glasübergangstemperatur (Tg) ist eine spezifische Materialeigenschaft von Kunststoffen. Sie bezeichnet die Temperatur, bei der amorphe oder teilkristalline Polymere vom festen Zustand in den flüssigen Zustand übergehen, wobei eine deutliche Änderung physikalischer Kenngrößen, z. B. der Härte und der Elastizität eintritt.

Besonders bevorzugt ist, dass das erste Material und das zweite Material eine Glasübergangstemperatur von mindestens 121°C aufweisen und vorzugsweise das dritte Material eine Glasübergangstemperatur von unter 121 °C aufweist. Besonders bevorzugt ist ferner, dass das dritte Material eine Glasübergangstemperatur von über 50°C, insbesondere über 55°C, über 60°C, über 65°C, über 70°C, über 75°C oder über 80°C aufweist. Ferner ist bevorzugt, dass das erste und das zweite Material eine Glasübergangstemperatur von über 125°C, insbesondere über 130°C, über 140°C, über 150°C, über 160°C, über 170°C oder über 180°C aufweisen.

Die Glasübergangstemperaturen des ersten Materials und des zweiten Materials sind vorzugsweise mindestens 5°C höher, insbesondere mindestens 10°C höher, mindestens 15°C höher, mindestens 20°C höher, mindestens 25°C höher, mindestens 30°C höher, mindestens 25°C höher, mindestens 30°C höher, mindestens 40°C höher, mindestens 50°C höher, mindestens 60°C höher, mindestens 70°C höher oder mindestens 80°C höher als die Glasübergangstemperatur des dritten Materials.

Eine besonders bevorzugte Materialpaarung ergibt sich durch Ausbildung der Kopfplatte und des Behälters aus Polyamid, Polycarbonat, Polymethylpenten oder Polypropylen und Ausbildung des Rührelements und/oder der Rührwelle aus Polystyrol. Diese Materialien weisen Eigenschaften auf, die sie für den Einsatz sowohl in der Zellkultur als auch in der Mikrobiologie geeignet machen. Gleichzeitig unterscheiden sich die für Kopfplatte und Behälter genannten Materialien von dem für das Rührelement und/oder die Rührwelle genannten Material in ihrer Temperaturbeständigkeit derart, dass das Polyamid, Polycarbonat, Polymethylpenten oder Polypropylen einen Dekontaminierungsprozess, insbesondere mittels Dampfsterilisation, im Wesentlichen unbeschadet übersteht, während das Polystyrol bei der Dampfsterilisation zum Schmelzen gebracht wird, sodass die Rührwelle bzw. das Rührelement nicht erneut verwendet werden können.

Die bisher beschriebenen Aspekte können einzeln oder in beliebiger Kombination zum Einsatz kommen. Insbesondere können die im Folgenden beschriebenen Weiterbildungen mit Einwegbioreaktoren nach jedem einzelnen der genannten Aspekte oder beliebigen Kombinationen dieser Aspekte kombiniert werden.

Eine bevorzugte Ausführungsform sieht vor, dass die Kopfplatte und der Behälter miteinander verklebt sind. Alternativ ist bevorzugt, dass die Kopfplatte und der Behälter miteinander verschweißt sind, insbesondere mittels Ultraschallschweißen oder Infrarotschweißen. Eine solche unlösbare Verbindung von Kopfplatte und Behälter durch Kleben oder Schweißen verringert die Kontaminationsgefahr durch eine Doppelverwendung oder ein ungewolltes Öffnen des Bioreaktors. Insbesondere das Ultraschallschweißen wird als Herstellungsverfahren bevorzugt, da es eine hohe Prozesssicherheit und damit einhergehende zuverlässige Verbindung von Kopfplatte und Behälter mit einer schnellen und einfachen Herstellung mit reduziertem Fertigungsaufwand verbindet.

Die Kopfplatte ist einstückig ausgebildet. Dies reduziert einerseits die Anzahl der Teile im Reaktionsraum, wodurch Toträume und Spalte vermieden werden, und andererseits kann dadurch auf zusätzliche Arbeitsschritte beim Zusammenbau bzw. der Montage des Einwegbioreaktors verzichtet werden. Besonders bevorzugt ist die Herstellung der Kopfplatte im Spritzgussverfahren.

Ferner ist die Herstellung der Kopfplatte aus Polyamid, Polycarbonat, Polymethylpenten oder Polypropylen bevorzugt. Diese Werkstoffe haben den Vorteil, einerseits die Anforderungen für einen Einsatz sowohl in der Zellkultur als auch für Anwendungen in der Mikrobiologie zu erfüllen und gleichzeitig eine hohe Temperaturbeständigkeit aufzuweisen.

Eine weitere bevorzugte Ausführungsform des Einwegbioreaktors sieht vor, dass die Kopfplatte auf ihrer Innenseite mehrere Tauchrohre aufweist, die in den Reaktionsraum ragen. Diese Tauchrohre sind vorzugsweise als hohle Hülsen ausgebildet und können verschiedene Instrumente, Sensoren, oder Leitungen, insbesondere flexible Schläuche, aufnehmen. Die Tauchrohre sind vorzugsweise als formstabile Rohre ausgebildet. Vorzugsweise korrespondieren die Tauchrohre auf der Innenseite der Kopfplatte mit Anschlüssen auf der Außenseite der Kopfplatte, sodass durch die Anschlüsse und die Tauchrohre Medien oder Elemente in den Reaktionsraum ein- bzw. aus diesem herausgeführt werden können. Vorzugsweise ragen die Tauchrohre, zumindest ein Teil der Tauchrohre, so weit in den Reaktionsraum hinein, dass sie bei einer bestimmungsgemäßen Verwendung des Einwegbioreaktors in einen im Reaktionsraum befindlichen Inhalt, beispielsweise eine Flüssigkeit, eintauchen. Daher die Bezeichnung Tauchrohre. Dies hat den Vorteil, dass auf der Innenseite der Kopfplatte keine weiteren Rohre oder Schläuche angeschlossen werden müssen, sondern die bereits an der Kopfplatte ausgebildeten Tauchrohre, die einstückig mit der gesamten Kopfplatte ausgebildet sind, verwendet werden können. Dies hat den Vorteil, dass im Herstellungsprozess Montageschritte eingespart werden können und ferner die Anzahl einzelner Baueinheiten, zwischen denen Spalte oder Toträume entstehen können, im Reaktionsraum verringert wird.

Ferner ist eine Ausführung des Einwegbioreaktors bevorzugt, bei dem die Kopfplatte eine Ausbuchtung zur Aufnahme des Lagers aufweist. Eine solche nach außen gerichtete Ausbuchtung der Kopfplatte ist bevorzugt, um das Lager der in dem Reaktionsraum angeordneten Rührwelle aufzunehmen und somit den nutzbaren Reaktionsraum durch die Anordnung des Lagers nur möglichst geringfügig zu verkleinern. Ferner kann an einer solchen Ausbuchtung auf der Außenseite der Kopfplatte einen Rotationsantrieb für das Rührwerk angeordnet werden. Insbesondere ist bevorzugt, dass die Ausbuchtung keine Öffnung in der Kopfplatte aufweist, was insbesondere bei einer Magnetkopplung, insbesondere bei der zuvor beschriebenen stirnseitigen Magnetkopplung, zwischen Rotationsantrieb und Rührwelle bevorzugt ist.

Insbesondere ist bevorzugt, dass der Bioreaktor eine weiter unten beschriebene Kopfplatte oder eine ihrer Fortbildungen aufweist. Zu den besonderen Vorteilen, Ausführungsvarianten und Ausführungsdetails dieser Kopfplatte und ihrer Fortbildungen wird auf unten folgende Beschreibung zu den entsprechenden Merkmalen der Kopfplatte und ihrer Fortbildungen verwiesen.

Ferner ist es bevorzugt, dass der Einwegbioreaktor ein Lagergehäuse aufweist, das einen Lagerraum zur Aufnahme des Lagers innerhalb des Reaktionsraums abgrenzt. Insbesondere in Kombination mit der zuvor beschriebenen Ausbuchtung in der Kopfplatte hat die hier beschriebene Ausführungsform den Vorteil, dass der Lagerraum teilweise, vorzugsweise überwiegend, im Bereich der Ausbuchtung der Kopfplatte angeordnet ist und so ein unterhalb der Kopfplatte angeordneter Teil des Reaktionsraums im Wesentlichen für die bestimmungsgemäße Verwendung des Bioreaktors zur Verfügung steht. Besonders bevorzugt ist es, dass das Lagergehäuse den Lagerraum gegenüber dem Reaktionsraum abgrenzt, beispielsweise durch eine Gleitlagerbuchse.

Das Lagergehäuse ist vorzugsweise an der Innenseite der Kopfplatte lösbar befestigt oder unlösbar mit der Innenseite der Kopfplatte verbunden. Eine lösbare Befestigung des Lagergehäuses kann beispielsweise eine Rastverbindung, Clipverbindung oder Verschraubung sein. Eine unlösbare Verbindung des Lagergehäuses mit der Innenseite der Kopfplatte kann beispielsweise durch Kleben oder Schweißen, insbesondere Ultraschallschweißen, hergestellt werden.

Gemäß einem vierten Aspekt wird eine biotechnologische Vorrichtung beschrieben, umfassend einen zuvor beschriebenen Einwegbioreaktor gemäß seiner verschiedenen Aspekte oder Fortbildungen, und einen Rotationsantrieb mit einem magnetischen Antriebsabschnitt, der derart angeordnet und ausgebildet ist, dass er in einer axialen Richtung mit dem magnetischen Abschnitt der Rührwelle magnetisch koppelbar ist.

Vorzugsweise besteht der magnetische Antriebsabschnitt aus einem Kompositwerkstoff mit einer Kunststoffmatrix und einem magnetischen Material oder weist einen solchen Kompositwerkstoff auf.

Ferner ist bevorzugt, dass der Komposit- bzw. Zweikomponentenwerkstoff mit einer Magnetkomponente auf ein stirnseitiges Antriebselement des Rotationsantriebs aufgespritzt ist.

In einer besonders bevorzugten Ausführungsform weist der magnetische Antriebsabschnitt einen Querschnitt in einer Ebene orthogonal zu einer Rotationsachse auf, und weist über den Großteil dieses Querschnitts, vorzugsweise über den gesamten Querschnitt, eine magnetische Kraftwirkung für eine magnetische Kopplung in axialer Richtung mit dem magnetischen Abschnitt der Rührwelle auf.

Eine solche Ausgestaltung eines vorzugsweise flächigen, zusammenhängenden Querschnitts des Bereichs mit magnetischer Kraftwirkung des Antriebsabschnitts kann insbesondere durch Spritzgießen eines Kompositwerkstoffs mit einer Kunststoffmatrix und einem magnetischen Material in einer Spritzgießform erzielt werden, die ihrerseits einen Magneten aufweist, um das magnetischen Material des Kompositwerkstoffs beim Spritzgießen auszurichten. Der Querschnitt des magnetischen Antriebsabschnitts mit magnetischer Kraftwirkung ist vorzugsweise kreisförmig, ellipsenförmig oder rechteckig. Innerhalb dieses Querschnitts des magnetischen Antriebsabschnitts mit magnetischer Kraftwirkung sind vorzugsweise Segmente unterschiedlicher Polung ausgebildet. Beispielsweise können die Segmente ein sternförmiges Muster oder ein Muster mit tortenstückförmigen Segmenten bilden. Das Muster der Segmente des magnetischen Antriebsabschnitts ist vorzugsweise auf das Muster der Segmente des magnetischen Abschnitts der Rührwelle abgestimmt.

Eine solche Ausgestaltung hat mehrere Vorteile: Zum einen kann gegenüber einer Ausbildung mit mehreren, ringförmig angeordneten Stabmagneten ein höheres Drehmoment übertragen werden, was insbesondere erforderlich ist zur Erzielung hoher Drehzahlen von über 1500 rpm, insbesondere bis zu 2000 rpm, bis zu 3000 rpm oder darüber, die insbesondere für Anwendungen in der Mikrobiologie erforderlich sind. Dabei kann gleichzeitig die erforderliche Querschnittsfläche des Rotationsantriebs und damit der erforderliche Bauraum auf der Kopfplatte gering gehalten werden. Ferner kann durch die Ausbildung von bestimmten Polsegmentmustern eine spezifische Passung von Bioreaktoren zu einem entsprechend ausgebildeten Rotationsantrieb realisiert und damit die Prozesssicherheit erhöht werden, da nur Bioreaktoren mit passender Polsegmentierung von einem Rotationsantrieb angetrieben werden können. Dies ist vorteilhaft, um den Antrieb mit dem gewünschten Drehmoment und der gewünschten Drehzahl sicherzustellen, da Drehmoment und insbesondere erzielte Drehzahl nur bei mechanisch an einen Rotationsantrieb gekoppelten Rührwerken überwacht werden, nicht hingegen bei magnetischen Kopplung von Rührwerk und Rotationsantrieb. Eine abweichende Drehzahl bei Verwendung beispielsweise eines Rotationsantriebs mit zu geringer Leistung kann jedoch den Kultivierungsprozess negativ beeinflussen.

Zu den Vorteilen, Ausführungsvarianten und Ausführungsdetails dieser biotechnologischen Vorrichtung und ihrer Fortbildungen wird auf die vorangegangene Beschreibung zu den entsprechenden Vorrichtungsmerkmalen des Einweg-Bioreaktors verwiesen.

Die eingangs genannte Aufgabe wird gelöst durch eine Kopfplatte nach Anspruch 1 Diese Kopfplatte ist insbesondere geeignet für einen zuvor beschriebenen Einwegbioreaktor, seine verschiedenen Aspekte und Fortbildungen. Die Kopfplatte umfasst eine Innenseite und eine der Innenseite gegenüberliegende Außenseite, wobei die Innenseite mehrere Tauchrohre aufweist und die Außenseite mehrere Anschlüsse aufweist, und wobei die Kopfplatte einstückig ausgebildet ist.

Ein solche einstückige Ausbildung einer Kopfplatte, die gleichzeitig auf ihrer Außenseite mehrere Anschlüsse und auf ihrer Innenseite mehrere Tauchrohre aufweist, hat den Vorteil eines besonders hohen Integrationsgrades. Dadurch wird die Montage eines Einwegbioreaktor deutlich vereinfacht, da die Tauchrohre Leitungen, wie z.B. Schläuche, ersetzen, die bei bekannten Kopfplatten an auf der Innenseite vorhandene Anschlüsse montiert werden müssen. Durch die einstückige Ausbildung wird auch die Anzahl von Teilen im Reaktionsraum und damit die Anzahl von Spalten und Toträumen reduziert.

Besonders bevorzugt ist, dass die Tauchrohre eine Länge aufweisen, so dass die Tauchrohre bei einem Mindestfüllvolumen eines Einwegbioreaktors, mit dem die Kopfplatte verwendet wird, in den Inhalt bzw. die Kulturbrühe eintauchen. Insbesondere ist eine Länge der Tauchrohre von mindestens 85 Prozent eines Durchmessers der Kopfplatte bevorzugt. Als Durchmesser ist hier beispielsweise der Durchmesser einer im Querschnitt kreisförmigen Kopfplatte zu verstehen. Bei einer Kopfplatte mit einem elliptischen oder eckigen Querschnitt ist unter Durchmesser eine Ausdehnung der Kopfplatte in einer ihrer beiden Haupterstreckungsrichtungen zu verstehen.

Die Tauchrohre weisen eine Länge von über 50 Prozent des Durch-messers der Kopfplatte auf. Besonders bevorzugt ist eine Länge der Tauchrohre von mindestens 75 Prozent, mindestens 80 Prozent, mindestens 85 Prozent, mindestens 90 Prozent oder mindestens 95 Prozent des Durchmessers der Kopfplatte. Ferner ist bevorzugt, dass die Länge der Tauchrohre mindestens dem Durchmesser der Kopfplatte entspricht. Ferner ist eine Länge der Tauchrohre bevorzugt, die größer als das 1,1-fache, größer als das 1,2-fache, größer als das 1,3-fache, größer als das 1,4-fache, größer als das 1,5-fache, größer als das 1,6-fache, größer als das 1,7-fache, größer als das 1,8-fache, größer als das 1,9-fache oder größer als das 2-fache des Durchmessers der Kopfplatte ist.

Die Kopfplatte weist vorzugsweise mindestens drei, insbesondere mindestens fünf Tauchrohre auf.

Die Tauchrohre weisen vorzugsweise einen Innendurchmesser von weniger als 8 Millimeter, insbesondere weniger als weniger als 7 Millimeter, weniger als 6 Millimeter, weniger als 5 Millimeter, weniger als 4,8 Millimeter, weniger als 4,75 Millimeter, weniger als 4,7 Millimeter, weniger als 4,5 Millimeter, weniger als 4,3 Millimeter, weniger als 4 Millimeter, weniger als 3,5 Millimeter, weniger als 3 Millimeter, weniger als 2,5 Millimeter, weniger als 2 Millimeter, weniger als 1,5 Millimeter oder weniger als 1 Millimeter auf.

Bevorzugt ist ferner, dass zumindest zwei, vorzugsweise mehrere, der Tauchrohre einer Kopfplatte unterschiedliche Innendurchmesser aufweisen. Bevorzugt ist ferner, dass zumindest zwei, vorzugsweise mehrere, der Tauchrohre einer Kopfplatte unterschiedliche Längen aufweisen. Diese Ausbildungen sind bevorzugt, um die Tauchrohre auf unterschiedliche Verwendungen anzupassen, um einen flexiblen, breiten Einsatzbereich eines Einwegbioreaktors mit einer solchen Kopfplatte zu schaffen.

Besonders bevorzugt ist, dass die Kopfplatte im Spritzgussverfahren hergestellt ist.

Vorzugsweise ist die Kopfplatte aus Polyamid, Polycarbonat, Polymethylpenten oder Polypropylen bevorzugt. Diese Werkstoffe haben den Vorteil, einerseits die Anforderungen für einen Einsatz sowohl in der Zellkultur als auch für Anwendungen in der Mikrobiologie zu erfüllen und gleichzeitig eine hohe Temperaturbeständigkeit aufzuweisen.

Ferner weist die Kopfplatte vorzugsweise eine Ausbuchtung zur Aufnahme eines Lagers eines Rührwerks auf. Dies ist insbesondere bevorzugt, wenn die Kopfplatte als Kopfplatte eines zuvor beschriebenen Einwegbioreaktors mit einer Magnetkopplung zwischen Rührwerk und Rotationsantrieb verwendet werden soll.

Besonders bevorzugt ist, dass sich der Durchmesser von zumindest einem der Tauchrohre an seinem der Kopfplatte abgewandten Ende verjüngt. Vorzugsweise ist dieses sich verjüngende Tauchrohr und/oder eines oder mehrere der anderen Tauchrohre an seinem der Kopfplatte abgewandten Ende verschlossen und ferner vorzugsweise an diesem verschlossenen Ende mit einer Öffnung versehen, wobei diese Öffnung vorzugsweise einen Durchmesser aufweist, der geringer ist als der Innendurchmesser des Tauchrohrs. Dies ist insbesondere bevorzugt, wenn ein solches Tauchrohr als Begasungsrohr eingesetzt werden soll.

Die Tauchrohre weisen vorzugsweise eine Wandstärke von unter 3 Millimeter auf, insbesondere eine Wandstärke unter 2 Millimeter, unter 1,5 Millimeter oder unter 1 Millimeter.

Zumindest einer, vorzugsweise zwei der Anschlüsse auf der Außenseite der Kopfplatte weisen ein Gewinde auf, vorzugsweise ein Innengewinde. Auf diese Weise können an den Anschlüssen Schraubverbindungen hergestellt werden, ohne dass dafür zunächst weitere Anschlusselemente an der Kopfplatte angebracht werden müssen.

Gemäß einem weiteren Aspekt der Erfindung wird die eingangs genannte Aufgabe gelöst durch ein Verfahren zum Herstellen einer Kopfplatte für einen Einwegbioreaktor nach Anspruch 12. Zu den Vorteilen, Ausführungsvarianten und Ausführungsdetails dieses Verfahrensaspektes der Erfindung und seiner Fortbildungen wird auf die vorangegangene Beschreibung zu den entsprechenden Vorrichtungsmerkmalen des Einweg-Bioreaktors und der Kopfplatte verwiesen.

### Beschreibung bevorzugter Ausführungsbeispiele der Erfindung

Einige beispielhafte Ausführungsformen der Erfindung werden im Folgenden anhand der beiliegenden Figuren beschrieben. Es zeigen:
- Fig. 1A:: eine dreidimensionale Ansicht einer beispielhaften Ausführungsform eines Einwegbioreaktors;
- Fig. 1B:: eine Seitenansicht des Einwegbioreaktors gemäß Fig. 1A;
- Fig. 1C:: eine Schnittdarstellung des Einwegbioreaktors gemäß Fig. 1A entlang der Schnittebene A-A in Fig. 1C;
- Fig. 1D:: einen vergrößerten Ausschnitt aus Fig. 1C;
- Fig. 1E:: eine alternative Ausgestaltung des magnetischen Abschnitts;
- Fig. 2A:: eine dreidimensionale Ansicht einer biotechnologischen Vorrichtung mit einem Einwegbioreaktor, einer Anschlussvorrichtung und einer Temperiereinrichtung;
- Fig. 2B:: eine Seitenansicht der biotechnologischen Vorrichtung gemäß Fig. 2A;
- Fig. 2C:: eine Draufsicht auf die biotechnologische Vorrichtung gemäß Fig. 2A;
- Fig. 2D:: eine Schnittdarstellung der biotechnologischen Vorrichtung gemäß Fig. 2A entlang der Schnittebene A-A in Fig. 2C;
- Fig. 2E:: einen vergrößerten Ausschnitt aus Fig. 2D;
- Fig. 2F:: eine alternative Ausgestaltung des magnetischen Abschnitts der Rührwelle und des magnetischen Rotationsabschnitts des Rotationsantriebs;
- Fig. 3:: ein paralleles Bioreaktorsystem;
- Fig. 4A:: den Einwegbioreaktor gemäß Fig. 1A mit Angabe eines Vergrößerungsausschnitts B;
- Fig. 4B:: den vergrößerten Ausschnitt B aus Fig. 4A; ;
- Fig. 5A:: eine dreidimensionale Ansicht einer Kopfplatte;
- Fig. 5B:: eine Seitenansicht der Kopfplatte gemäß Fig. 5A; und
- Fig. 5C:: eine Draufsicht auf die Kopfplatte gemäß Fig. 5A.

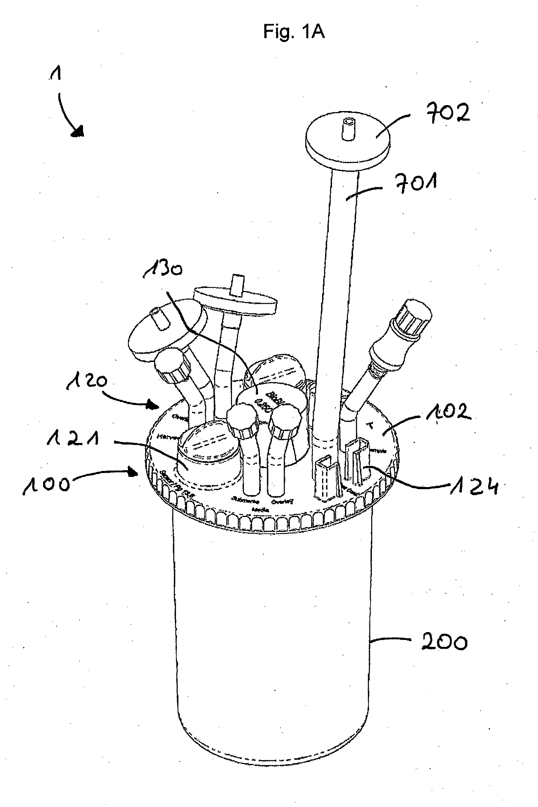

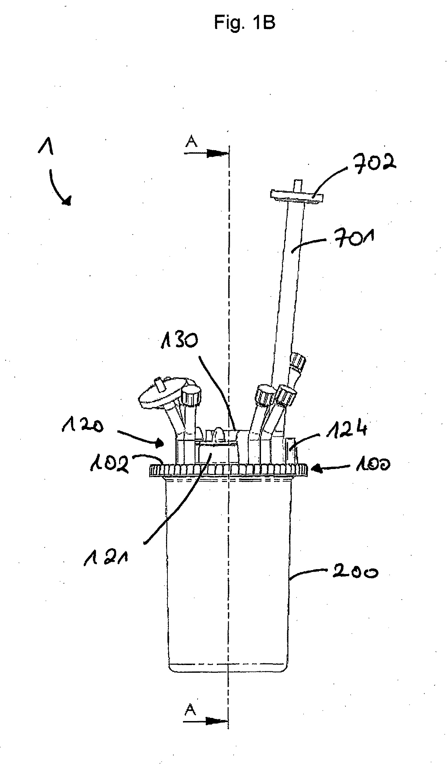

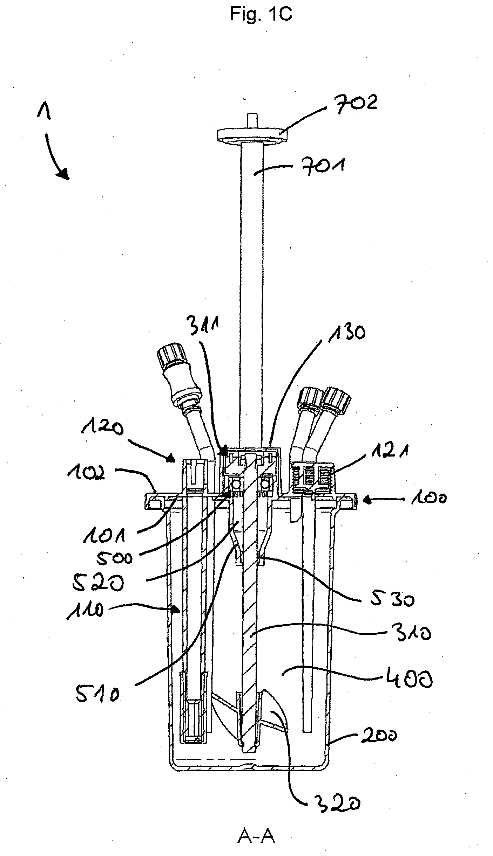

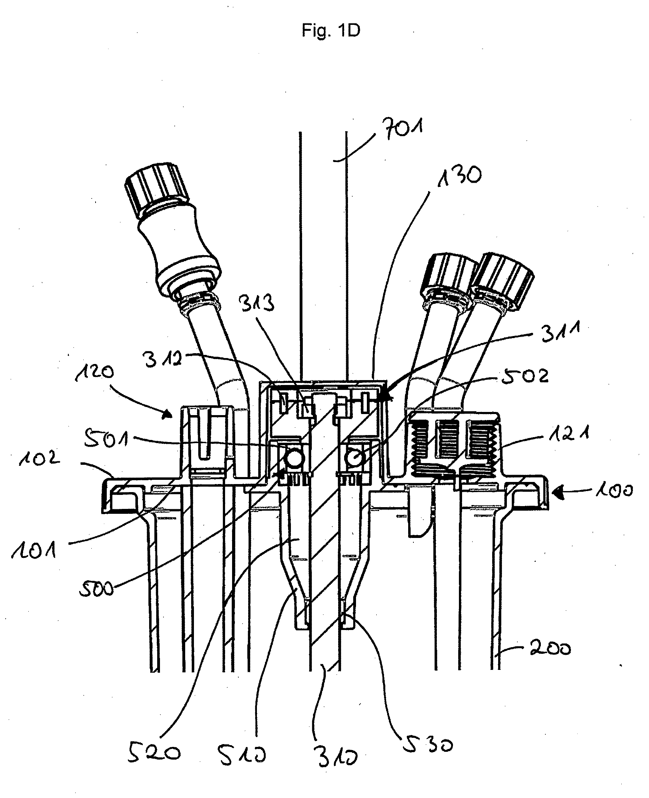

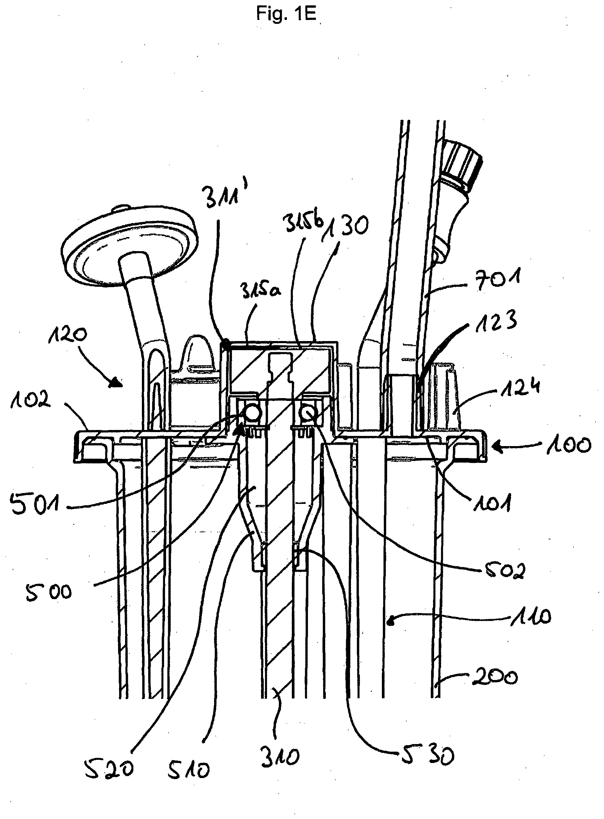

Die Figuren 1 bis 5 stellen beispielhafte Ausführungsformen g und deren Anwendung dar. Gleiche oder ähnliche Elemente werden in den Figuren mit den gleichen Bezugszeichen bezeichnet.

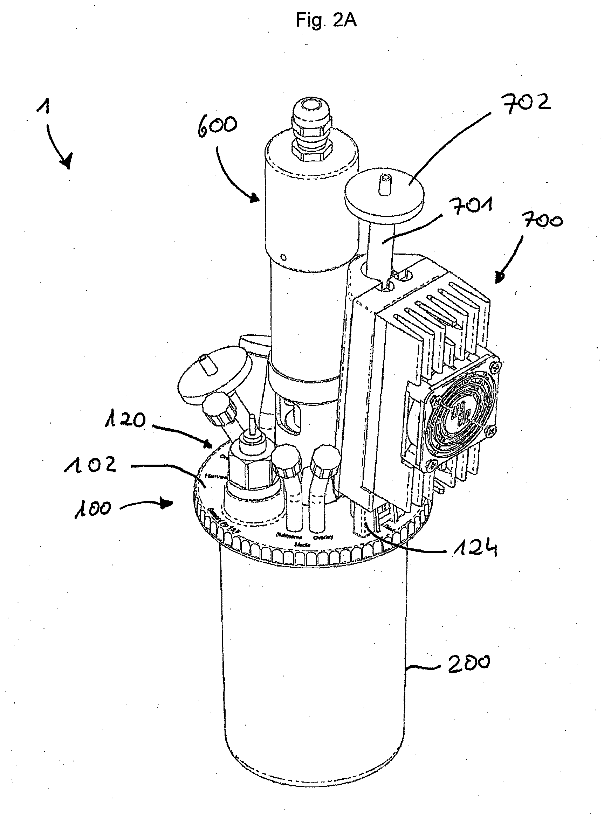

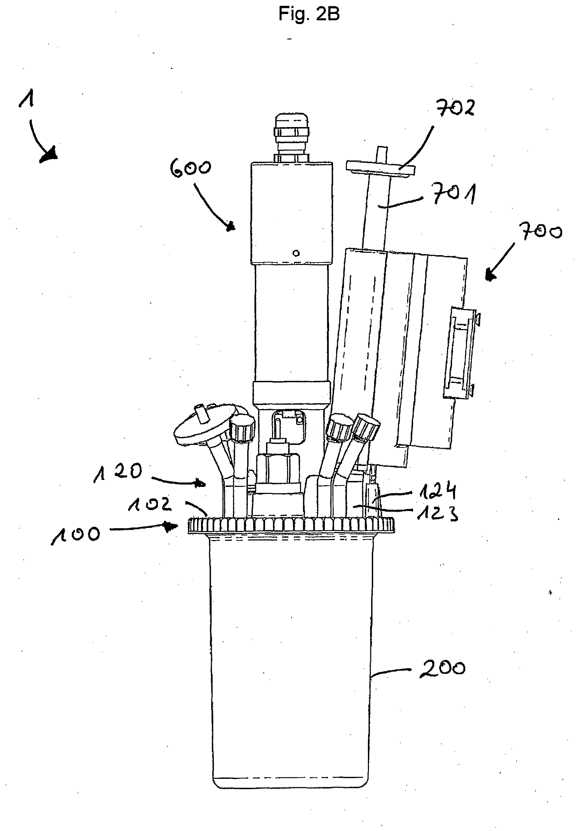

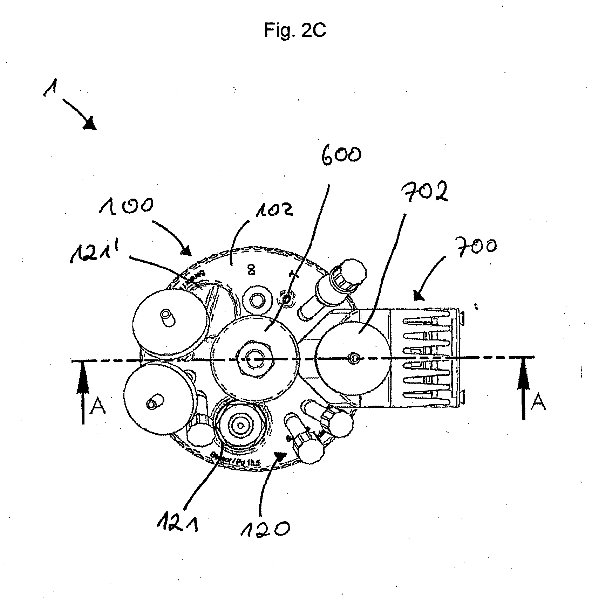

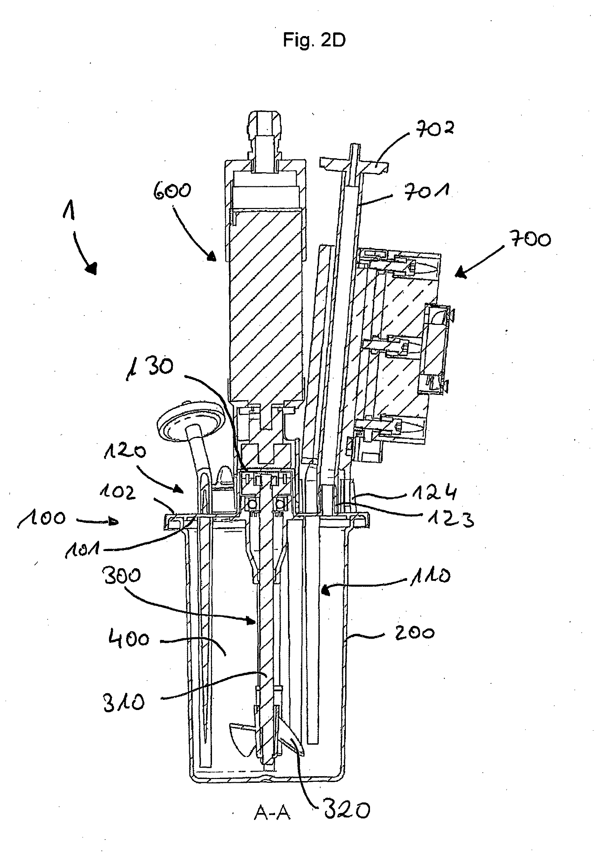

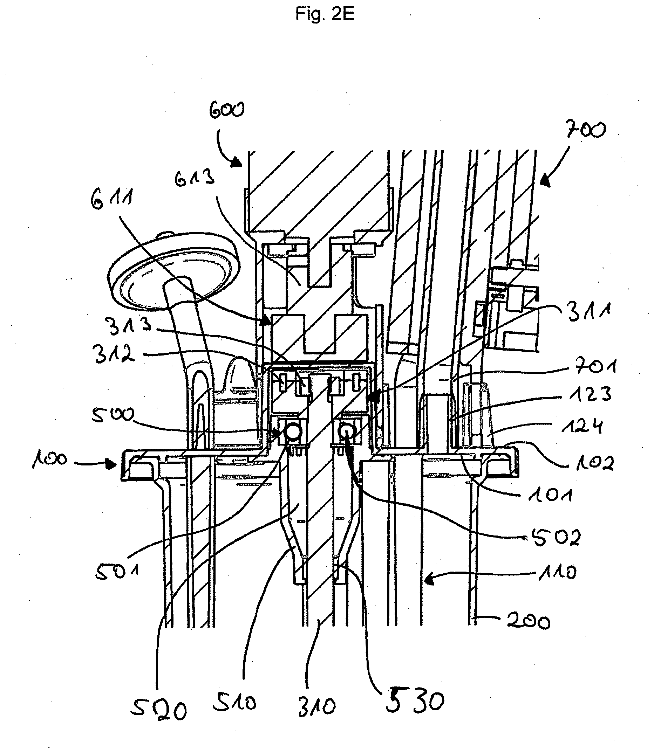

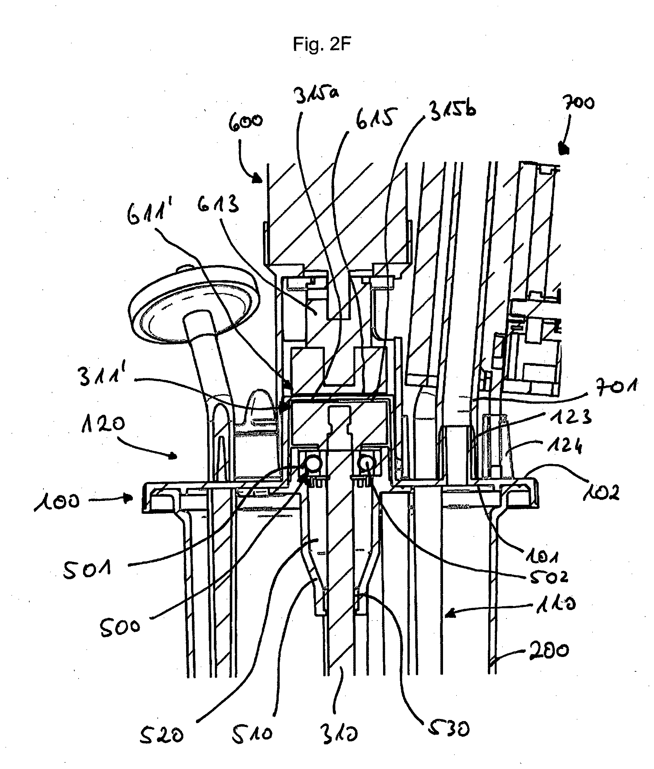

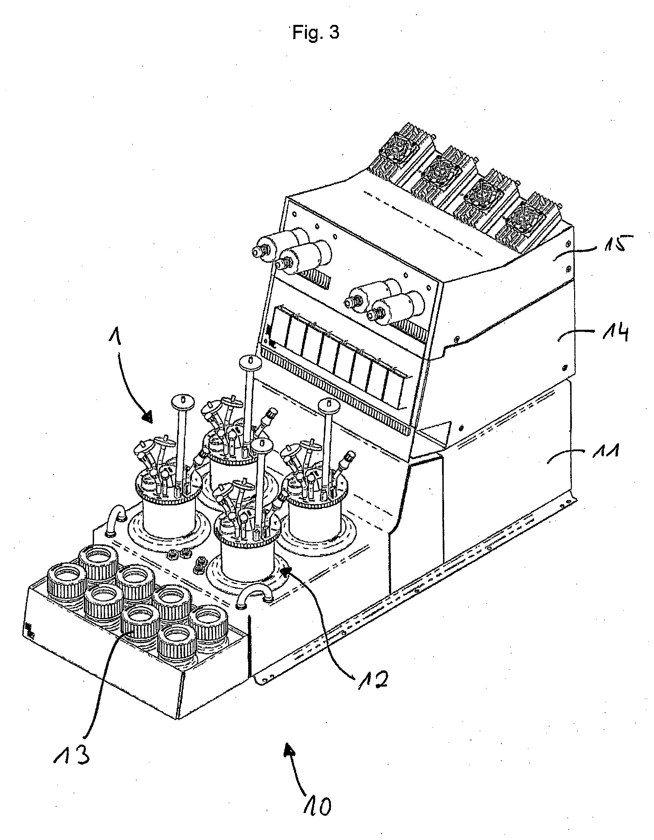

In den Figuren 1A,B,C,D,E und 2A,B,C,D,E,F ist ein Einwegbioreaktor 1 bzw. eine biotechnologische Vorrichtung mit einem Einwegbioreaktor, einer Anschlussvorrichtung und einer Temperiereinrichtung zur Verwendung in einem in Fig. 3 dargestellten parallelen Bioreaktorsystem 10 für die Anwendung in der Zellkultur und/oder in der Mikrobiologie dargestellt. Das in Fig. 3 gezeigte parallele Bioreaktorsystem 10 weist einen Basisblock 11 mit vier darin angeordneten Aufnahmen 12 auf, in die jeweils ein Bioreaktor 1 lösbar eingesetzt werden kann. In dem Basisblock 11 ist vorzugsweise eine Temperatursteuerung angeordnet, die ausgebildet ist, die in den Aufnahmen 12 angeordneten Einwegbioreaktoren 1 nach Bedarf zu heizen oder zu kühlen. Angrenzend an den Basisblock 11 ist eine Anordnung mit Behältern 13 ausgebildet. Ferner weist der Basisblock 11 eine Stapelfläche auf, auf der zwei Funktionsblöcke 14, 15 abnehmbar in einer Stapelformation angeordnet sind und beispielsweise als Ablage- und Anzeigestation oder Pumpenstation ausgebildet sind, beispielsweise um die für den Betrieb der Bioreaktoren notwendigen Fluide zu- oder abzuführen. Ein solches paralleles Bioreaktorsystem 10 hat den Vorteil einer geringen Standfläche und einer hohen Skalierbarkeit, da mehrere dieser parallelen Bioreaktorsysteme 10 mit jeweils vier Einwegbioreaktoren 1 nebeneinander angeordnet werden können. Die Einwegbioreaktoren 1 haben den Vorteil, in einem solchen parallelen Bioreaktorsystem für die Anwendung in der Zellkultur und/oder der Mikrobiologie wie wiederverwendbare Bioreaktoren eingesetzt werden zu können.

Der Einwegbioreaktor 1 weist eine Kopfplatte 100, einen formstabilen Behälter 200 und ein Rührwerk 300 auf. Die Kopfplatte 100 und der Behälter 200 schließen einen Reaktionsraum 400 ein. Die Kopfplatte 100 hat eine dem Reaktionsraum zugewandte Innenseite 101, an der mehrere Tauchrohre 110 angeordnet sind, die in den Reaktionsraum 400 ragen. Auf einer dem Reaktionsraum 400 abgewandten Außenseite 102 der Kopfplatte 100 sind mehrere Anschlüsse 120 angeordnet.

Das Rührwerk 300 weist eine Rührwelle 310 mit einer Rotationsachse und einem Rührelement 320 auf. Das Rührelement 320 ist hier mit um 45° geneigten Flügeln ausgebildet, beispielsweise als Pitch Blade Impeller. Alternativ kann beispielsweise auch mindestens ein Rushton-Impeller als Rührelement eingesetzt werden. Das Rührelement 320 ist drehsteif an der Rührwelle 310 befestigt, sodass bei einer Rotation der Rührwelle 310 auf das Rührelement 320 mitrotiert.

Die Kopfplatte 100 und der Behälter 200 sind vorzugsweise aus Polyamid ausgebildet und mittels Ultraschallschweißen miteinander unlösbar verbunden. Das Rührwerk 300, insbesondere die Rohrwelle 310 und/oder das Rührelement 320, sind vorzugsweise aus Polystyrol ausgebildet. Polystyrol weist eine geringere Temperaturbeständigkeit auf als Polyamid, sodass bei einer Heißdampfsterilisation des Einwegbioreaktors 1 das Rührwerk 300 unbrauchbar gemacht wird und damit eine Wiederverwendung des Einwegbioreaktors 1 ausgeschlossen ist.

Die Rührwelle 310 ist um die Rotationsachse drehbar in einem Lager 500 gelagert. Das Lager 500 ist in einer Ausbuchtung 130 der Kopfplatte 100 angeordnet. Ein Lagergehäuse 510 grenzt einen Lagerraum 520 in dem Reaktionsraum 400 ab. Die Rührwelle 310 ist durch eine Gleitlagerbuchse 530 aus dem Lagergehäuse 510 herausgeführt. Vorzugsweise kann das gesamte Lagergehäuse aus einem Material bestehen, das für eine Gleitlagerung geeignet ist. Dies hat den Vorteil, einer Einzelteilreduktion und eines hohen Integrationsgrades, was sich auch vorteilhaft in der Herstellung und Montage auswirkt. Das Lager 500 ist als Wälzlager ausgebildet und ist vorzugsweise ein Polymerkugellager 501 mit einem Käfig aus Polyethylen, Polypropylen, Polyvinylidenfluorid, Polyetheretherketon oder Polytetrafluorethylen sowie Glaskugeln 502.

Das Rührwerk 300 und das Lager 500 sind vollständig in dem Reaktionsraum 400 angeordnet. Wie insbesondere in Fig. 1D,E und 2E,F zu erkennen ist, weist die Rührwelle 310 einen magnetischen Abschnitt 311, 311' auf, der in einer axialen Richtung mit einem Rotationsantrieb 600 magnetisch koppelbar ist. Der magnetische Abschnitt 311, 311' der Rührwelle 310 ist stirnseitig, d.h. in axialer Richtung, mit einem Rotationsantrieb 600 magnetisch gekoppelt. Der Rotationsantrieb 600 ist im Wesentlichen zylinderförmig ausgebildet und hat eine Querschnittsfläche, die im Wesentlichen der Querschnittsfläche der Ausbuchtung 130 auf der Kopfplatte 100 entspricht. Ein Antriebselement 613 treibt einen magnetischen Antriebsabschnitt 611, 611' an. Die in Fig. 1D,E und 2E,F gezeigte stirnseitige, axiale magnetische Kopplung weist den Vorteil eines besonders geringen Platzbedarfs für den Antrieb auf der Kopfplatte auf.

In der in den Fig. 1D, 2E gezeigten Variante ist am Ende der Rührwelle 310 ist ein magnetischer Abschnitt 311 angeordnet, in dem, vorzugsweise in einer ringförmigen Anordnung, Magnete 312 angeordnet sind. In dem in Fig. 1D und 2E gezeigten Ausführungsbeispiel ist der magnetische Abschnitt 311 mit den darin angeordneten Magneten 312 über eine Sechskantmutter 313 drehsteif mit der Rührwelle 310 verbunden. In dem magnetischen Antriebsabschnitt 611 des Rotationsantriebs können ebenfalls Magnete angeordnet sein, deren Anordnung vorzugsweise auf die Anordnung der Magnete 312 des magnetischen Abschnitts 311 der Rührwelle 310 ausgerichtet ist.

In einer alternativen, besonders bevorzugten Ausführungsform gemäß der Fig. 1E, 2F weist der magnetische Abschnitt 311' einen Querschnitt in einer Ebene orthogonal zur Rührwelle 31 auf, und weist über den Großteil dieses Querschnitts, vorzugsweise über den gesamten Querschnitt, eine magnetische Kraftwirkung für eine magnetische Kopplung in axialer Richtung mit einem Rotationsantrieb 600 auf. Der Querschnitt des magnetischen Abschnitts 311' mit magnetischer Kraftwirkung ist hier vorzugsweise kreisförmig ausgebildet und weist vorzugsweise Segmente 315a,b unterschiedlicher Polung auf, die beispielsweise ein sternförmiges Muster oder ein Muster mit tortenstückförmigen Segmenten bilden.

Vorzugsweise weist auch der magnetische Antriebsabschnitt 611' des Rotationsantriebs 600 einen Querschnitt in einer Ebene orthogonal zu einer Rotationsachse auf, und weist über den Großteil dieses Querschnitts, vorzugsweise über den gesamten Querschnitt, eine magnetische Kraftwirkung für eine magnetische Kopplung in axialer Richtung mit dem magnetischen Abschnitt 311' der Rührwelle 310 auf.

Der Querschnitt des magnetischen Antriebsabschnitts 611' mit magnetischer Kraftwirkung ist hier vorzugsweise kreisförmig ausgebildet und weist vorzugsweise Segmente 615 unterschiedlicher Polung auf, die beispielsweise ein sternförmiges Muster oder ein Muster mit tortenstückförmigen Segmenten bilden. Das Muster der Segmente 615 des magnetischen Antriebsabschnitts 611' ist vorzugsweise auf das Muster der Segmente 315a,b des magnetischen Abschnitts 311' der Rührwelle 310 abgestimmt.

Der magnetische Abschnitt 311' und/oder der magnetische Antriebsabschnitt 611' ist bzw. sind vorzugsweise aus einem Komposit- oder Zweikomponenten-Werkstoff, insbesondere einem Magnet-Polymer-Gemisch, ausgebildet und ferner vorzugsweise im Spritzgussverfahren hergestellt. Der magnetischen Abschnitt 311' und/oder der magnetische Antriebsabschnitt 611' kann bzw. können als separate Teile, die an der Rührwelle 310 bzw. dem Antriebselement 613 angeordnet und dort, ggf. lösbar, befestigt werden, ausgebildet sein.

Besonders bevorzugt ist eine einstückige Ausbildung des magnetischen Abschnitts 311' mit der Rührwelle 310, vorzugsweise durch Aufspritzen eines ein Komposit- oder Zweikomponenten-Werkstoffs, insbesondere eines Magnet-Polymer-Gemischs, auf ein Ende der Rührwelle 310. Besonders bevorzugt ist ferner eine einstückige Ausbildung des magnetischen Antriebsabschnitts 611' mit einem Antriebselement 613, vorzugsweise durch Aufspritzen eines ein Komposit- oder Zweikomponenten-Werkstoffs, insbesondere eines Magnet-Polymer-Gemischs, auf das Antriebselement 613.

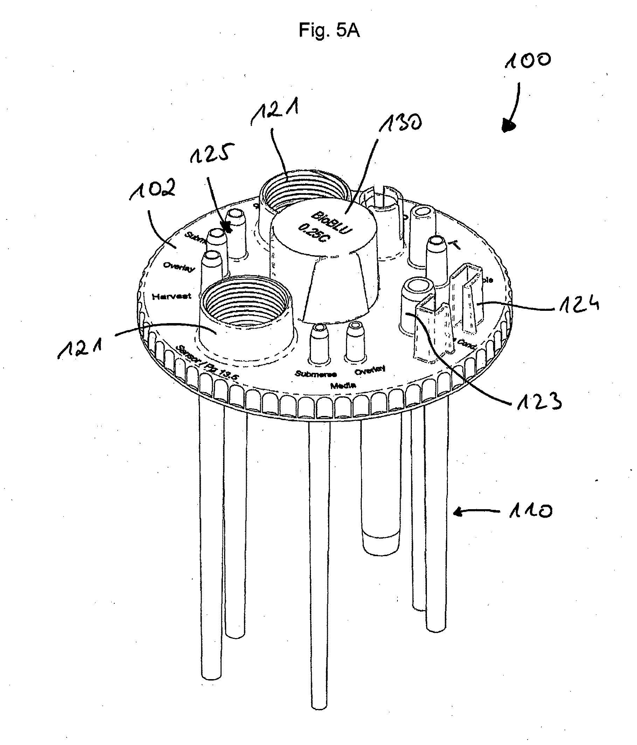

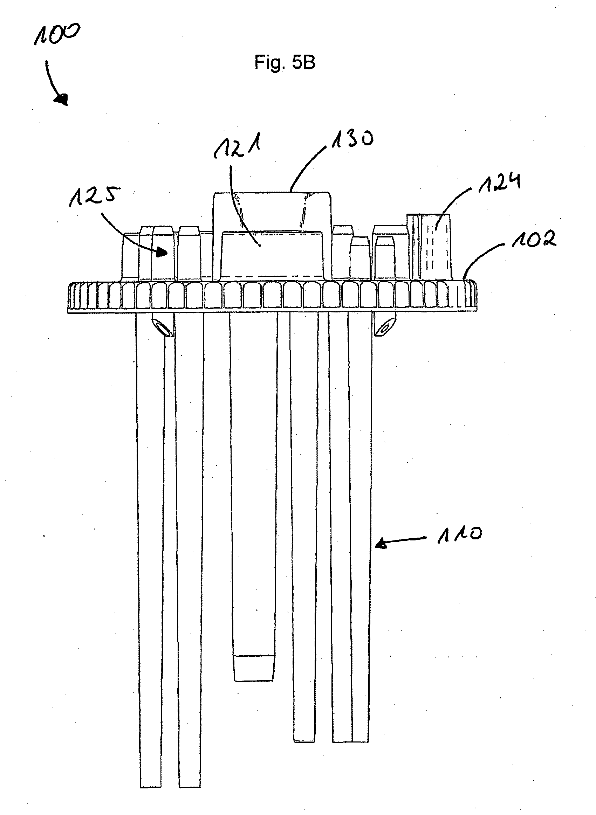

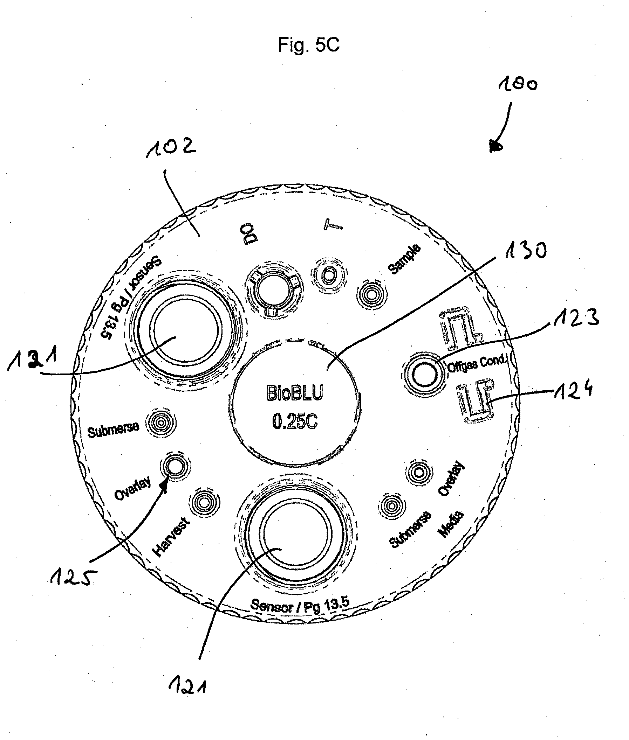

Die insbesondere in Fig. 5A,B,C dargestellte Kopfplatte 100 ist vorzugsweise einstückig ausgebildet und im Spritzgussverfahren aus vorzugsweise Polyamid hergestellt einschließlich der auf der Außenseite angeordneten Anschlüsse 120 und der auf der Innenseite angeordneten Tauchrohre 110. Die Tauchrohre 110 korrespondieren mit einem Teil der Anschlüsse 120, sodass durch die korrespondierenden Anschlüsse 120 Instrumente, Sensoren, Leitungen, wie z.B. Schläuche durch die Tauchrohre 110 in den Reaktionsraum ein- bzw. ausgeführt werden können. Die Anschlüsse 120 dienen dazu, um die für den Reaktionsprozess notwendigen Stoffe bereitzustellen und/oder Stoffe aus dem Reaktionsraum 400 abzuführen, z.B. beim Betrieb entstehende Gase. Die Anschlüsse 120 können auch als Overlay und die Tauchrohre 110 als Submers bezeichnet werden.

Der Anschluss 123 dient beispielsweise dazu, mit einen Abgasschlauch 701 verbunden zu werden, an dessen Ende wiederum ein Abgas-Sterilfilter 702 angeordnet ist. Ein durch den Abgasschlauch 701 abgeführte Gasstrom kann beispielsweise durch eine weitere Vorrichtung behandelt, vorzugsweise eine Temperiereinrichtung, werden. Der Sterilfilter 702 dient dazu, das Abgas vor seinem Austritt zu filtern. Neben dem Anschluss 123 für einen Abgasschlauch 701 ist ein Anschlussschlitz 124 durch zwei U-förmige Profile gebildet, die insbesondere dafür geeignet sind, ein Abgaskühlelement 700 aufzunehmen. Das Abgaskühlelement 700 kann insbesondere als Temperierelement ausgebildet sein, wie es in der parallelen Anmeldung der Anmelderin vom selben Tage mit dem Titel 'Vorrichtung für eine sterile Einweg-Fluidleitung eines Einwegbioreaktors und Verfahren zum Behandeln eines Fluidstroms" beschrieben ist. Ein solches Kühlelement 700 dient dazu, das Abgas im Schlauch 701 zu kühlen und darin mitgeführte Flüssigkeit zu kondensieren, die dann, vorzugsweise durch Gravitation, in den Reaktionsraum 400 zurückgeführt werden kann und damit einerseits im Reaktionsraum wieder zur Verfügung steht und andererseits den Sterilfilter 702 nicht verstopft.

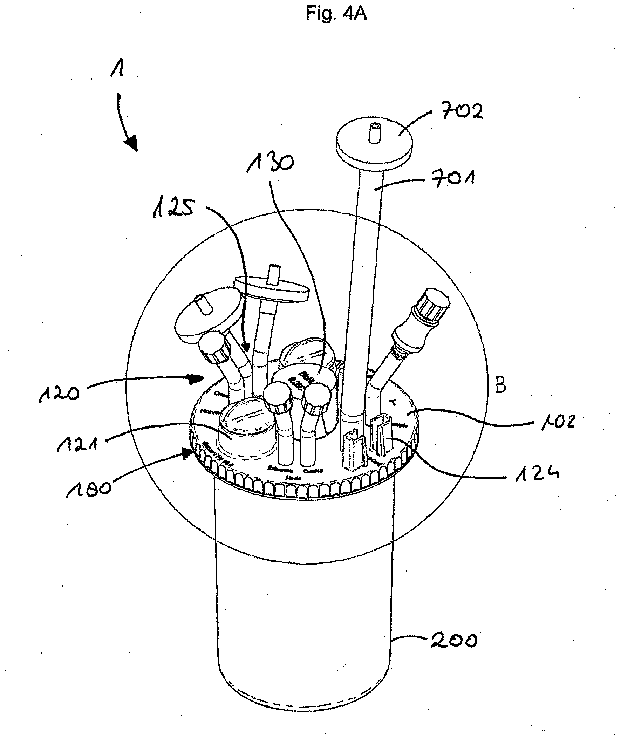

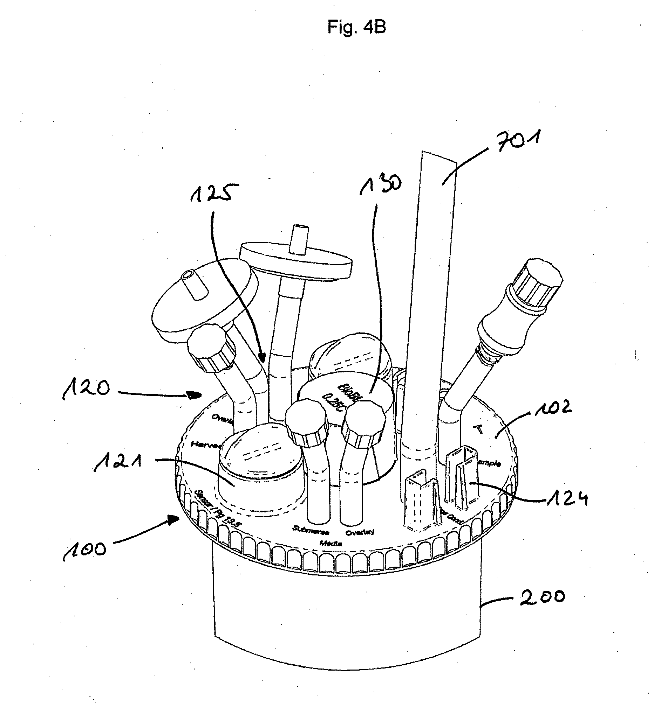

Die Anschlüsse 120 können beispielsweise als Schraubanschlüsse 121 mit einem Innengewinde, als Klemmanschlüsse 122 oder als kegelförmige Anschlüsse 125 ausgebildet sein. Die in Fig. 5A,B,C dargestellte Anordnung von Anschlüssen 120 und Tauchrohren 110 ermöglicht eine hohe Flexibilität, sodass ein Einwegreaktor mit einer solchen Kopfplatte 100 für eine Vielzahl von Anwendungen sowohl in der Zellkultur als auch in der Mikrobiologie geeignet ist. Nicht benötigte Anschlüsse 120 können für die Anwendung verschlossen werden, wie dies beispielsweise für die beiden Schraubanschlüsse 121 in Fig. 1A und 4A,B dargestellt ist. Ferner sind z.B. in Fig. 5A,B,C drei zwischen den beiden Schraubanschlüssen 121 liegende kegelförmige Anschlüsse 125 zu erkennen, an denen in den Fig. 4A,B Schläuche mit aufgesteckt sind. In der in Fig. 2C dargestellten Variante ist der hintere der beiden Schraubanschlüsse 121' mit einer Kappe verschlossen, wohingegen der vordere der beiden Schraubanschlüsse 121 ein Funktionselement trägt. An die Anschlüsse 120 können beispielsweise pH- oder DO-(Dissolved Oxygen)-, Temperatur-oder andere Sensoren angeordnet und vorzugsweise über die Tauchrohre 110 in den Reaktionsraum eingeführt werden. Die beiden Schraubanschlüsse 121 sind vorzugsweise als PG 13,5 Gewinde ausgebildet.

Eine besonders bevorzugte Kombination von Anschlüssen 120 einer Kopfplatte 100 beinhaltet zwei als PG 13,5 Gewinde ausgebildete Schraubanschlüsse, Gasanschlüsse für Kopfraum- und Unter-Wasser-(bzw. Unter-Medien-)Gaszufuhr, einen Abgasanschluss sowie eine Steckverbindung für eine Abgaskühlung, einen Beprobungsanschluss mit einem Beprobungsventil, beispielsweise einem swabable valve, einen Medienanschluss, zwei Tauchrohre, einen Anschluss für einen Widerstandstemperaturfühler (resistance temperature detector (T oder RTD)) und einen Gelöstsauerstoff(DO)-Sensoranschluss mit einer permeablen Membran. Eine besonders bevorzugte Kombination von Anschlüssen 120 auf der Kopfplatte 100 ist in den Fig. 5A,B,C dargestellt.

Mit dem Einwegbioreaktor 1 verwendete Schläuche und Anschlussmaterialien, die mit Reaktionsmedien in Berührung kommen können, sind vorzugsweise aus Materialien ausgebildet, die nach der United States Pharmacopeia (USP) Klasse VI zertifiziert sind, wie beispielsweise Polystyrol, Polycarbonat, Polyamid oder Silikon. Die einzusetzenden Schläuche sind vorzugsweise flexible Schläuche aus thermoplastischen Elastomeren.

Die in der vorstehenden Beschreibung, den Ansprüchen und den Zeichnungen offenbarten Merkmale der Erfindung können sowohl einzeln als auch in beliebiger Kombination für die Verwirklichung der Erfindung in ihren verschiedenen Ausführungen von Bedeutung sein.

## Patentansprüche

1. Kopfplatte (100) für einen Einwegbioreaktor (1), insbesondere zur Verwendung in einem, vorzugsweise parallelen, Bioreaktorsystem (10), für die Anwendung in der Zellkultur und/oder der Mikrobiologie,
die Kopfplatte (100) umfassend eine Innenseite (101) und eine der Innenseite (101) gegenüberliegende Außenseite (102),
wobei die Außenseite (102) mehrere Anschlüsse (120) aufweist und die Innenseite (101) mehrere Tauchrohre (110) aufweist, die einstückig mit der gesamten Kopfplatte (100) ausgebildet sind, und wobei die Tauchrohre eine Länge von über 50 Prozent eines Durchmessers der Kopfplatte aufweisen.

2. Kopfplatte (100) nach dem vorhergehenden Anspruch,
**dadurch gekennzeichnet, dass** die Tauchrohre (110) eine Länge aufweisen, die größer ist als der ein Durchmesser der Kopfplatte (100).

3. Kopfplatte (100) nach mindestens einem der vorhergehenden Ansprüche,
**dadurch gekennzeichnet, dass** die Tauchrohre (110) einen Innendurchmesser von weniger als 5 Millimeter und/oder eine Wandstärke von weniger als 3 Millimeter aufweisen.

4. Kopfplatte (100) nach mindestens einem der vorhergehenden Ansprüche,
**dadurch gekennzeichnet, dass** zumindest zwei der Tauchrohre (110) unterschiedliche Innendurchmesser aufweisen.

5. Kopfplatte (100) nach mindestens einem der vorhergehenden Ansprüche,
**dadurch gekennzeichnet, dass** zumindest zwei der Tauchrohre (110) unterschiedliche Längen aufweisen.

6. Kopfplatte (100) nach mindestens einem der vorhergehenden Ansprüche,
**dadurch gekennzeichnet, dass** die Kopfplatte (100) im Spritzgussverfahren hergestellt ist.

7. Kopfplatte (100) nach mindestens einem der vorhergehenden Ansprüche,
**dadurch gekennzeichnet, dass** die Kopfplatte (100) eine Ausbuchtung zur Aufnahme eines Lagers (500) eines Rührwerks (300) aufweist.

8. Kopfplatte (100) nach mindestens einem der vorhergehenden Ansprüche,
**dadurch gekennzeichnet, dass** sich der Durchmesser von zumindest einem der Tauchrohre (110) an seinem der Kopfplatte (100) abgewandten Ende verjüngt.

9. Kopfplatte (100) nach dem vorhergehenden Anspruch,
**dadurch gekennzeichnet, dass** das sich verjüngende Tauchrohr (110) und/oder eines oder mehrere der anderen Tauchrohre an seinem der Kopfplatte (100) abgewandten Ende verschlossen und vorzugsweise an diesem verschlossenen Ende mit einer Öffnung versehen ist.

10. Kopfplatte (100) nach mindestens einem der vorhergehenden Ansprüche,
**dadurch gekennzeichnet, dass** zumindest einer der Anschlüsse (120) auf der Außenseite der Kopfplatte (100) ein Innengewinde aufweist.

11. Verfahren zum Herstellen einer Kopfplatte (100) für einen Einwegbioreaktor (1), insbesondere zur Verwendung in einem, vorzugsweise parallelen, Bioreaktorsystem (10), für die Anwendung in der Zellkultur und/oder der Mikrobiologie, insbesondere einer Kopfplatte einem der Ansprüche 1-10,
umfassend die Schritte:
Spritzgießen einer Kopfplatte (100) umfassend eine Innenseite (101) und eine der Innenseite (101) gegenüberliegende Außenseite (102), wobei die Außenseite (102) mehrere Anschlüsse (120) aufweist und die Innenseite (101) mehrere Tauchrohre (110) aufweist, die einstückig mit der gesamten Kopfplatte (100) ausgebildet sind, und wobei die Tauchrohre eine Länge von über 50 Prozent eines Durchmessers der Kopfplattem aufweisen.

## Claims

1. Head plate (100) for a single-use bioreactor (1), in particular for use in a, preferably parallel, bioreactor system (10) for use in cell culture and/or microbiology,
the head plate (100) having an inner side (101) and an outer side (102) opposite the inner side (101),
wherein the outer side (102) has a plurality of connections (120) and the inner side (101) a plurality of dip tubes (110), which form integral parts of the overall head plate (100), and wherein the dip tubes (110) have a length of more than 50 per cent of a diameter of the head plate (100).

2. Head plate (100) according to the preceding claim, **characterised in that** the dip tubes (110) have a length that is greater than a diameter of the head plate (100).

3. Head plate (100) according to at least one of the preceding claims, **characterised in that** the drip tubes (110) have an internal diameter of less than 5 millimetres and/or a wall thickness of less than 3 millimetres.

4. Head plate (100) according to at least one of the preceding claims, **characterised in that** at least two of the dip tubes (110) have different internal diameters.

5. Head plate (100) according to at least one of the preceding claims, **characterised in that** at least two of the dip tubes (110) have different lengths.

6. Head plate (100) according to at least one of the preceding claims, **characterised in that** the head plate (100) is manufactured by injection moulding.

7. Head plate (100) according to at least one of the preceding claims, **characterised in that** the head plate (100) has a dent for accommodating a bearing (500) of a stirrer (300).

8. Head plate (100) according to at least one of the preceding claims, **characterised in that** the diameter of at least one of the dip tubes (110) tapers at its end opposite the head plate (100).

9. Head plate (100) according to the preceding claim, **characterised in that** the tapering dip tube (110) and/or one or more of the other dip tubes is sealed at its end opposite the head plate (100) and preferably at this sealed end is provided with an opening.

10. Head plate (100) according to at least one of the preceding claims, **characterised in that** at least one of the connections (120) on the outer side of the head plate (100) has an inside thread.

11. Method for producing a head plate (100) for a single-use bioreactor (1), in particular for use in a, preferably parallel, bioreactor system (10) for use in cell culture and/or microbiology, in particular a head plate according to any one of Claims 1 to 11,
comprising the steps:
injection moulding of a head plate (100) having an inner side (101) and an outer side (102) opposite the inner side (101), wherein the outer side (102) has a plurality of connections (120) and the inner side (101) a plurality of dip tubes (110), which form integral parts of the overall head plate (100), and wherein the dip tubes (110) have a length of more than 50 per cent of a diameter of the head plate (100).

## Revendications

1. Plaque supérieure (100) pour un bioréacteur jetable (1), en particulier destinée à être utilisée dans un système de bioréacteur (10), de préférence parallèle, pour application dans la culture cellulaire et/ou dans la microbiologie,
laquelle plaque supérieure (100) comprend un côté intérieur (101) et un côté extérieur (102) faisant face au côté intérieur (101),
sachant que le côté extérieur (102) présente plusieurs raccordements (120) et que le côté intérieur (101) présente plusieurs tubes plongeurs (110), qui sont réalisés d'un seul tenant avec l'intégralité de la plaque supérieure (100), et sachant que les tubes plongeurs (110) présentent une longueur supérieure à 50 % d'un diamètre de la plaque supérieure (100).

2. Plaque supérieure (100) selon la revendications précédente,
**caractérisée en ce que** les tubes plongeurs (110) présentent une longueur, qui est supérieure au diamètre de la plaque supérieure (100).

3. Plaque supérieure (100) selon au moins l'une quelconque des revendications précédentes,
**caractérisée en ce que** les tubes plongeurs (110) présentent un diamètre intérieur inférieur à 5 millimètres et/ou une épaisseur de paroi inférieure à 3 millimètres.

4. Plaque supérieure (100) selon au moins l'une quelconque des revendications précédentes,
**caractérisée en ce qu'**au moins deux des tubes plongeurs (110) présentent des diamètres intérieurs différents.

5. Plaque supérieure (100) selon au moins l'une quelconque des revendications précédentes,
**caractérisée en ce qu'**au moins deux des tubes plongeurs (110) présentent des longueurs différentes.

6. Plaque supérieure (100) selon au moins l'une quelconque des revendications précédentes,
**caractérisée en ce que** la plaque supérieure (100) est fabriquée lors d'un procédé de moulage par injection.

7. Plaque supérieure (100) selon au moins l'une quelconque des revendications précédentes,
**caractérisée en ce que** la plaque supérieure (100) présente une protubérance servant à recevoir un palier (500) d'un mécanisme agitateur (300).

8. Plaque supérieure (100) selon au moins l'une quelconque des revendications précédentes,
**caractérisée en ce que** le diamètre d'au moins un des tubes plongeurs (110) se rétrécit au niveau de son extrémité opposée à la plaque supérieure (100).

9. Plaque supérieure (100) selon la revendication précédente,
**caractérisée en ce que** le tube plongeur (110) se rétrécissant et/ou un ou plusieurs des autres tubes plongeurs sont fermés au niveau de son/leur extrémité opposée à la plaque supérieure (100) et sont pourvus de préférence, au niveau de ladite extrémité fermée, d'une ouverture.

10. Plaque supérieure (100) selon au moins l'une quelconque des revendications précédentes,
**caractérisée en ce qu'**au moins un des raccordements (120) présente, sur le côté extérieur de la plaque supérieure (100), un filetage intérieur.

11. Procédé servant à fabriquer une plaque supérieure (100) pour un bioréacteur jetable (1), en particulier destinée à être utilisée dans un système de bioréacteur (10), de préférence parallèle, pour application dans la culture cellulaire et/ou dans la microbiologie, en particulier une plaque supérieure selon l'une quelconque des revendications 1 à 11,
comprenant les étapes suivantes consistant à :
mouler par injection une plaque supérieure (100) comprenant un côté intérieur (101) et un côté extérieur (102) faisant face au côté intérieur (101), sachant que le côté extérieur (102) présente plusieurs raccordements (120) et que le côté intérieur (101) présente plusieurs tubes plongeurs (110), qui sont réalisés d'un seul tenant avec l'intégralité de la plaque supérieure (100), et sachant que les tubes plongeurs (110) présentent une longueur supérieure à 50 % d'un diamètre de la plaque supérieure (100).
